# EUROPEAN PATENT APPLICATION

(11) **EP 4 216 126 A1**
(43) Date of publication of application: **26.07.2023**
(21) Application number: 23153197.1
(22) Date of filing: 25.01.2023
(51) Int. Cl.: G06Q 10/087, G06Q 10/0833, G06Q 50/28, A61G 12/00, A61B 50/13

(54) **SYSTEM AND METHOD FOR THE AUTHOMATIC MANAGEMENT AND TRACKING OF SURGICAL MATERIALS**

(30) Priority: 25.01.2022 IT 202200001211
(71) Applicant: Jaes Informatica Societa'Cooperativa, 42122 Reggio Emilia (RE) (IT)
(72) Inventor: Campani, Samuel, 42122 Reggio Emilia (RE) (IT)
(74) Representative: Petraz, Davide Luigi

(57) **Abstract**

The invention concerns a method and a system (10) for the automatic management of surgical materials (50) in a healthcare facility provided with operating theaters (200) and comprising one or more surgical trolleys (10), mobile to reach the operating theaters (200), and configured to house and transport the materials (50).

## Description

### FIELD OF THE INVENTION

The present invention concerns a system comprising at least one surgical trolley and a corresponding method for the automatic management and tracking of surgical materials.

The system and corresponding method can be used, by way of example but not only, for tracking surgical materials inside healthcare facilities where operating theaters suitable for hosting surgical procedures are provided.

### BACKGROUND OF THE INVENTION

In the healthcare sector, for example in hospitals, surgeries, or in general in any healthcare facility where operating theaters suitable for surgical operations are provided, it is necessary to manage the material essential to the operating theaters for each operation.

The surgical material can comprise sterile surgical instruments, kits of surgical instruments each comprising a specific and determinate number and type of surgical instruments, for example associated with a particular type of operation, material made of sterile RTF (Reusable Technical Fabrics), material made of NWF (Non-Woven Fabric), drugs and/or other medical devices.

In these structures, it is known that it is necessary to be able to automate the process of managing the surgical material in order to ensure, in the times and manner required, that all the material needed to carry out the single operation, or all the surgical operations scheduled in that theater throughout the day, is available at entry to the operating theater.

It is also known that, during operations, some surgical instruments may not actually be used and, at times, may be mistakenly thrown away or trace of them may be lost, something which constitutes a significant waste for healthcare facilities which, by optimizing the management thereof, would make significant economic savings. Surgical instruments and more generally the consumable materials needed in surgery are in fact expensive and delicate to handle.

Hence the need to have an inventory of surgical material that is always up-to-date.

Computerized trolleys are known, but these are usually configured to contain medicines, drugs and medications and to monitor their use in therapies on patients, mainly to ensure that the operators of the various shifts in the ward can keep track of the patient's treatment in order to prevent errors, in particular double or no administrations of drugs due to misunderstandings between colleagues who take turns in the different shifts.

Currently, trolleys to contain and transport surgical material are not computerized, nor are there any instruments to automate and optimize the logistic process to supply and make the material available to surgical theatres. An example of a trolley configured to contain medical instruments is described in U.S. Patent 2005/236940.

There is therefore a need to perfect a system comprising at least one surgical trolley and a corresponding method for the automatic management and tracking of surgical materials which can overcome at least one of the disadvantages of the state of the art.

One purpose of the present invention is to manage the process of supplying the surgical material efficiently and to bring operational advantages at least in terms of preparation time, correct assignment of the material, traceability of the operations and of the material, on the basis of a previously determined operating program.

Another purpose of the present invention is to allow to manage, as automatically as possible, the procedures for registering the material, both that used and that which remains unused, and to verify the need for material both in the storage step and in the use of the surgical material.

Another purpose of the present invention is to make available a system for the automatic management of surgical materials which allows the automatic collection of precise data for the attribution of costs even of the individual operation, thanks to which the healthcare facilities will be able to optimize procedures and reduce costs.

Another purpose is to guarantee the real-time updating of inventory information about the material contained in the surgical trolley and more generally in the system.

Another purpose is also to provide a system and a method which allow to know the identity of the operator who has loaded or unloaded the surgical materials into/from the trolley, also knowing which materials have been loaded or unloaded.

Yet another purpose is to allow rapid real-time geo-location of the trolleys inside the healthcare facility, also knowing in which operating theater the various surgical materials unloaded were used, in order to track them correctly.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, and to resolve the technical problem disclosed above in a new and original way, also achieving considerable advantages compared to the state of the prior art, a method for the automatic management and tracking of surgical or medical materials comprises the steps of:
- receiving a list of materials needed on the basis of the scheduling of one or more surgical operations of at least one operating theater in a pre-established time interval;
- through the automatic reading of identification codes located on the materials, verifying their removal from a materials warehouse and the subsequent placement in a mobile trolley dedicated to the operating theater;
- automatically updating the stock of the materials in the materials warehouse, by subtracting the quantities of materials removed from the current stock value in order to calculate a current stock;
- once the time interval has elapsed, detecting through identification means provided on board the trolley and/or in the operating theater which materials located in the trolley have remained unused;
- automatically updating the stock of the materials in the materials warehouse by adding to the current stock the quantities of unused materials after the latter have been detected by additional identification means present in the materials warehouse.

According to another aspect of the present invention, there is provided a system for the automatic management and tracking of surgical materials in a healthcare facility comprising one or more operating theaters, wherein the system comprises:
- one or more surgical trolleys configured to receive a plurality of surgical or medical materials and mobile in order to reach the operating theaters,
- identification codes located on the materials,
- identification means placed on board each of the trolleys, in each operating theater, in a materials warehouse and possibly in a pharmaceutical warehouse where the materials are stored,
- a central control unit configured to process the data received from the identification means in order to update the stock of materials present in the materials warehouse, also considering materials removed from the latter, placed in the trolleys and left unused at the end of a pre-established time interval.

Doing so achieves the advantage of managing the surgical material supply process efficiently, simplifying and speeding up the preparation of the surgical trolleys for each single surgical operation with the required materials.

An additional advantage is that, by calculating the stock of surgical material in the trolley, it is possible to keep track of the unused materials and reduce the likelihood that they are thrown away or lost, preventing waste and reducing material costs.

Advantageously, it is possible both to record the material actually used, and also to verify the need for surgical material, both in the storage phase as well as in the usage phase of the material itself.

It is also possible, by automatically collecting the data relating to the consumption of surgical material for each operation, to correctly allocate the costs even to the single operation.

It is also an advantage to be able to schedule the preparation and delivery of surgical materials for each individual operation on the basis of the surgical schedule for a pre-established time interval, for example daily, guaranteeing their availability within the times and in the manner required by the activity schedule of the operating theater.

Furthermore, it is possible to keep track of the materials, updated in real time, in particular of their quantity and their position within the healthcare facility, for example in the warehouses or in the operating theaters, thus reducing the process lead time.

Additional advantages are the automation of operations such as the reporting of all the material used for each single operation and the traceability of the materials treated, for example sterilized, and supplied, allowing to map on a computer system the phases of the logistics process along the entire movement circuit of the materials.

The work of hospital operators is also made faster and easier.

Advantageously, it is possible to simplify and possibly keep track of the operation of preparing the materials. By doing so, for example, only the instruments that have been correctly processed and are required for the specific surgical operation, or for the group of surgical operations, scheduled for the pre-established time period will be loaded into the trolley and taken to the operating theater, only removing from the warehouse the materials that are actually necessary to allow the operators to work efficiently without any waste of time and/or material.

### DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a schematic representation of a system for the automatic management and tracking of surgical materials according to the invention;
- fig. 2 is a schematic three-dimensional representation of an embodiment of a system for the automatic management and tracking of surgical materials according to the invention, comprising one or more surgical trolleys, for example a surgical trolley like that of fig. 4;
- fig. 3 is a block diagram of a method for the automatic management of surgical materials according to the present invention;
- fig. 4 is a schematic and simplified three-dimensional representation of a surgical trolley for the automatic management of surgical materials according to the present invention.

We must clarify that in the present description the phraseology and terminology used, as well as the figures in the attached drawings also as described, have the sole function of better illustrating and explaining the present invention, their function being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS OF THE PRESENT INVENTION

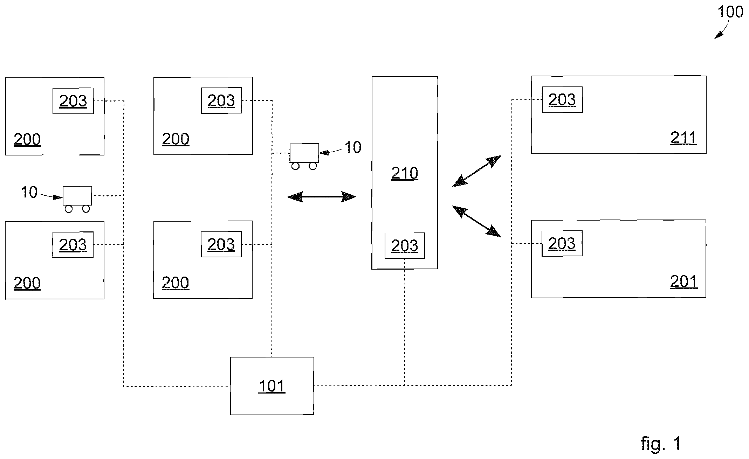

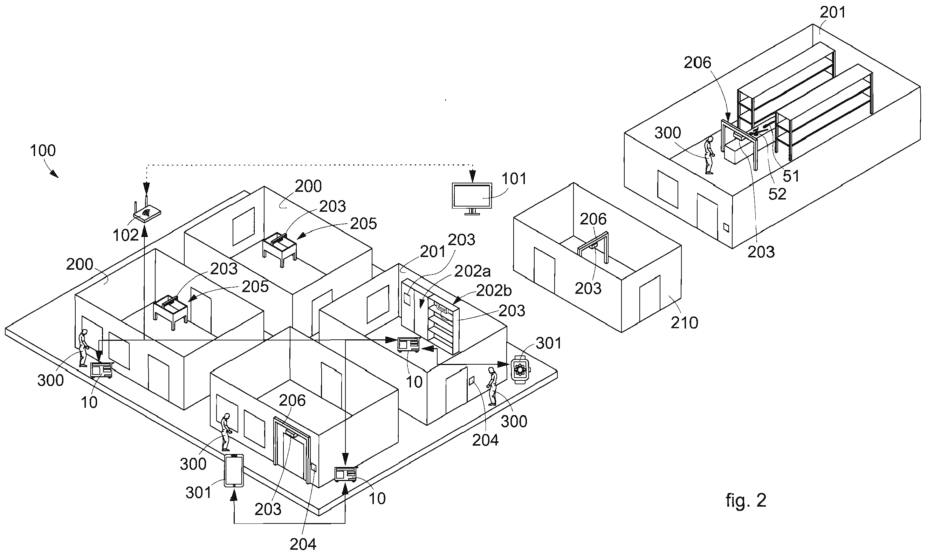

With reference to figs. 1 and 2, a system will be described below, indicated as a whole by reference number 100, for the automatic management and tracking of surgical materials or, more generally, of medical devices, in a healthcare facility.

These surgical materials can comprise both instruments and tools that can be reused several times after being subjected to accurate sterilization cycles after each use, such as scalpels, scissors, forceps or retractors, and also disposable consumables, such as sterile gauze and disposable syringes for example. In some embodiments, it is not excluded that such surgical materials may also comprise drugs or other medicaments to be administered to patients.

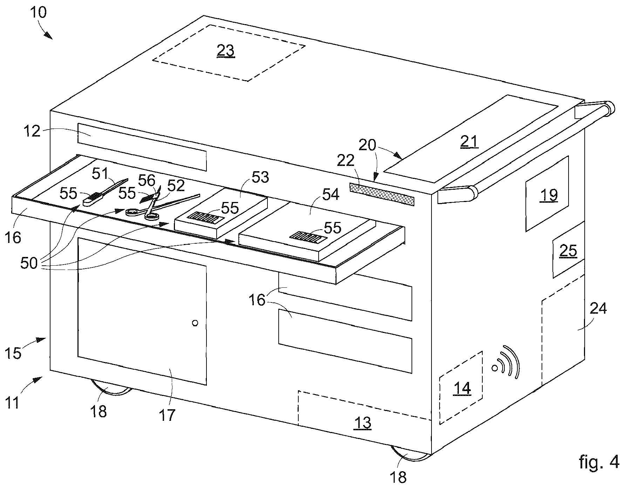

The system 100 comprises one or more surgical trolleys, hereafter also only "trolleys" for short, indicated with the reference number 10. For a better understanding of the present invention, we will now describe in detail an embodiment of a trolley 10 comprised in the system 100, with particular reference to fig. 4.

The trolley 10 comprises a containing structure 11 for housing and transporting the surgical materials 50, a device 12 for identifying the materials 50, a processing unit 13, a communication device 14.

The processing unit 13 can comprise a processing module, or CPU, a data storage module and auxiliary circuits. For example, the CPU can be any form of microcontroller, microprocessor, computer processor usable in the IT field. The storage module can be connected to the CPU and can consist of one or more commercially available memories, such as a random access memory (RAM), a read-only memory (ROM), a hard disk, mass storage, or any other form of digital storage whatsoever. The processing unit 13 can comprise auxiliary circuits, for example at least one of either cache circuits, power supply circuits, clock circuits, subsystems, user interface circuits and suchlike, which can be connected to the CPU in a conventional manner.

The processing unit 13 can comprise a management algorithm configured to perform, even automatically and/or in real time, at least the calculation of the stocks of materials 50 in the trolley 10. The algorithm can be stored in the storage module to command the CPU.

The trolley 10 can be configured at least to allow one or more of the following functions:
- detecting the identity of an operator 300 who performs the loading and unloading of the materials 50 into/from the trolley 10 itself,
- tracking the loaded and unloaded materials 50,
- providing information on the stocks of materials 50 in the trolley 10,
- allowing the localization of its position within the healthcare facility,
- providing the communication of data and information relating to the materials 50 contained in the trolley with a central control unit 101, external to the trolley 10.

In particular, the above functions can preferably take place in real time.

The containing structure 11 can comprise, in a known manner, a container 15 for housing the materials 50, comprising for example drawers 16, for example of the extractable type, compartments 17 with closable doors, an upper rest surface and/or suchlike.

The containing structure 11 can comprise, at the lower part, in a known manner, elements 18 for moving the trolley 10, such as wheels, possibly equipped with a braking system.

By way of example, fig. 4 shows a containing structure 11 with three drawers 16 of different formats and one compartment 17, in which a drawer 16 contains surgical instruments such as, merely by way of a non-limiting example, scalpels 51, scissors 52, a box of medicines 53, a sheet made of RTF 54.

The identification device 12 is configured to recognize the materials 50 contained inside it. By way of example, it can comprise one or more readers of identification codes 55, such as a video camera, bar code, QR code, NFC (Near Field Communication) code, RFID (Radiofrequency Identifier) code readers or suchlike, preferably an RFID reader. To this end, a unique identification code 55 among those listed above by way of example is disposed on each material 50.

The identification codes 55 can be integrated in the materials 50, for example they can be printed, created by means of laser processing, glued onto the materials 50 and/or they can be affixed onto labels 56, detachable or not, or by means of similar modes.

Advantageously, the identification codes 55 can be usable on metal components, resistant to high temperatures (up to 250°C) and can also be usable in sterile hospital environments.

The identification device 12 can also be configured to identify the operator 300, for example by reading an identification code thereof positioned on a personal badge or suchlike.

According to one variant, the trolley can comprise a detection device 19, dedicated exclusively to identifying the operator 300.

The communication device 14 can be configured for real-time communication with a central control unit 101, the functions of which will be explained in greater detail below in the context of the description of the system 100.

The communication device 14 can preferably be configured for automatic communication, at frequent and regular time intervals, but also for communication managed by an external user. Preferably, the communication device 14 is configured to perform real-time communication.

The identification device 12 and/or the processing unit 13 can communicate with the central control unit 101 by means of the communication device 14.

The communication device 14 can be configured to make the on-board data of the trolley 10 available for a possible multi-channel transmission request (Webserver, FTP - File Transfer Protocol, WebSocket or suchlike).

The communication device 14 can be any short-range and/or long-range wireless communication device whatsoever, such as for example Wi-Fi or Bluetooth systems, by means of 3G, 4G, 5G cellular technology, or similar and/or analogous systems. For this purpose, as a function of the technology adopted, the communication device 14 comprises the components necessary to allow correct communication, such as for example a SIM card, an antenna for data transmission/reception, etc.

The communication device 14 can possibly be configured to communicate with portable electronic devices 301 allocated to an operator 300, at least for sending data and information relating to the materials 50 contained in the trolley 10.

The trolley 10 can comprise one or more devices 20 for interfacing with the operator 300, selected from a monitor 21, for example of the touch-screen type, audio devices 22, such as microphones and/or speakers for example, a keyboard, a mouse and/or suchlike.

Advantageously, as an alternative to the automatic identification through the other detection device 19, the operator 300 can also identify him/herself manually, by entering his/her data, for example his/her name, a personal code and/or the identification number of the personal badge, by means of the interface devices.

The trolley 10 can comprise at least one telemetry, or localization, device 23 for detecting the position of the trolley 10 inside the healthcare facility.

The management algorithm can be configured to detect the position data and supply them to the central control unit 101, even in real time.

The telemetry device 23 can comprise extended range RFID-based technology, Bluetooth Low Energy (BLE) technology, centimeter Ultra-Wideband (UWB) systems, and/or similar technologies. The choice of the localization technology is, in a known manner, linked to the precision with which it is necessary to locate the trolley 10, to its energy autonomy and/or to the impact on the infrastructure of the healthcare facility and/or to similar aspects.

For example, the telemetry device 23 can interface with an identification code 204 identifying a position, for example an identification code 204 (fig. 2) which can be read by the telemetry device 23 and located in a plurality of positions in the healthcare facility, in correspondence with the access point of an operating theater 200, in a corridor or suchlike.

The trolley 10 can comprise an electric power supply device 24, for powering the electronic components, comprising a rechargeable battery or a non-rechargeable battery. The electric power supply device 24 can be sized to allow the trolley 10 an autonomy of at least 12 consecutive hours.

In particular, the management algorithm as above can be configured to manage consumption so that the autonomy of the trolley 10 is at least 12 consecutive hours.

The trolley 10 can comprise additional auxiliary detection devices 25, such as temperature sensors, speed sensors, displacement sensors, shock detectors of the trolley 10 or suchlike.

In particular, the processing unit 13 can be configured to collect, store and process the data measured by the auxiliary detection devices 25. Such data can be the result of point measurements or of statistical processing.

The system 100 (fig. 1) comprises one or more of the trolleys 10, which are mobile between one or more operating theaters 200 and a loading warehouse 210, in which the trolleys 10 are loaded with the materials 50. In some embodiments, the loading warehouse 210 can also integrate the function of station for sterilizing the materials 50 which need to be sterilized.

These materials 50 are stored in an instrument warehouse 201, configured to receive both the reusable instruments and also the disposable consumables in an orderly manner in special dedicated compartments, as will be explained in more detail below.

Some embodiments of the present invention also provide a pharmaceutical warehouse 211, configured to receive the drugs and medicines in an orderly manner in special drawers.

Preferably, the movement of the materials 50 between the warehouses 201 and 211 and the loading warehouse 210 is performed by one or more operators 300 in any known mode, which however does not provide to use trolleys 10. In fact, the trolleys 10 are configured to move only in a limited zone of the healthcare facility, characterized by high standards of hygiene and cleanliness, and therefore the trolleys 10 do not also directly reach the warehouses 201 and 211, which in practice are disposed in zones of the healthcare facility that are very remote from the operating block.

The system 100 comprises one or more fixed identification members 203, configured to detect the identification codes 55 located on the materials 50 and thus identify the different materials.

At least one of the fixed identification members 203 is disposed in the instrument warehouse 201. Other fixed identification members 203 are provided in each operating theater 200, in the loading warehouse 210 and in the pharmaceutical warehouse 211 (if provided).

The system 100 comprises a central control unit 101 configured to be operatively connected to the one or more trolleys 10 and the warehouses 201, 210, 211, as will be explained in more detail below.

The central control unit 101 can comprise a processing unit and a data storage unit. For example, the processing unit can be any form whatsoever of computer processor usable in the IT field. The storage unit can be connected to the processing unit, and it can be one or more commercially available memories, such as a random access memory (RAM), a read-only memory (ROM), a hard disk, mass storage or any other form of digital storage whatsoever. The central control unit 101 can comprise auxiliary circuits, for example at least one of either cache circuits, power supply circuits, clock circuits, subsystems, user interface circuits and suchlike.

In particular, the central control unit 101 can be configured as a processor capable of executing a management computer system or program.

The management computer system can be configured to carry out, even automatically and/or in real time, the operational analysis, the calculation of the stocks of materials 50 in the trolley 10 and/or in the warehouses 201, 210, 211, the localization of one or more trolleys, 10 and/or the analysis of the costs associated with the one or more trolleys 10. In order to do this, the central control unit 101 is configured to acquire, preferably in real time, the data detected by the devices 12, 19, 23, 25 provided on board the trolleys 10.

Moreover, the management computer system is configured to interface also with the fixed identification members 203 in order to track the movement of the materials 50 and therefore be able to calculate the stocks thereof also in the warehouses 201, 210 and 211.

The management computer system can also be configured for integration with the software of the healthcare facility.

The integration with the software of the healthcare facility, in particular aimed at allowing the retrieval of the requested information, can be implemented where possible by means of HL7 messaging using Web Services technology on Enterprise-bus, by means of alternative techniques for sharing information between systems or suchlike. The integration for the communication of surgical operations provides the exchange of information relating to the operations using shared coding (for example ICD 9 CM).

The software allows to manage the surgical scheduling of the operating blocks, for example by generating the daily lists of the operating sessions for each block, that is, the list of the surgical operations planned on a certain date. The request for an operation usually contains the basic information: ward, type of operation, estimated date, operating theater 200 in which the operation will be performed.

The central control unit 101 can be configured to acquire the above operating lists from the hospital software. Upon completion of the insertion of the operating list, whether it is entered directly into the computer system by a user or it comes automatically from the software of the healthcare facility, the computer system of the central control unit 101 can be configured to print the updated list, organize and re-organize the list according to different criteria of priority, severity, and suchlike.

The central control unit 101 can also be configured to acquire average theatre occupation times, material consumption, specific equipment, sterile surgical instruments, RTF KITs, NWF disposable material, drugs and any medical devices or surgical instruments necessary for the various types of operation and suchlike.

The central control unit 101 can therefore be configured to analyze the operating list according to the desired arrangements, such as by date and time of the request, by type of operation, by priority, by operating theater 200 and suchlike.

The central control unit 101 can be configured, starting from the requests selected by the operator 300 and/or from the information received from the software of the healthcare facility, to check the availability of the materials 50, possibly signaling their lack or shortage. For example, it can be configured to query the software of the healthcare facility, for example of the warehouses 201, 211, of the loading warehouse 210, of the laundry-room and suchlike, in order to acquire the data relating to the availability of the materials 50.

The central control unit 101 can also be configured to carry out the analysis of the requirement of the materials 50 for each surgical operation, as a function of the protocol defined by the hospital facility for each type of operation. Advantageously, in this way it is possible to program the processes of restocking the materials 50.

With reference to fig. 2, we will now describe an example embodiment of a system 100 according to the present invention.

In the example given, four operating theaters 200, the loading warehouse 210 and the instrument warehouse 201 are shown.

The system 100 also comprises communication devices 102 through which the central control unit 101 communicates at least with the one or more trolleys 10 and with the warehouses.

Through the communication devices 102, the central control unit 101 also communicates with the software of the healthcare facility. Such software can be comprised in one or more central units of the healthcare facility, such as on a central server, central units of operating theaters 200, of a sterilization station, of warehouses 201, 210, 211 and suchlike.

The communication devices 102 can comprise a dedicated broadband infrastructure, for example Wi-Fi, Wi-Fi 802.11b/g/n, through dedicated narrow band infrastructures but more penetrating at the RF level, such as LoRa and LoRaWAN solutions, or through the use of public networks of mobile operators, through 3G, 4G or 5G technologies or suchlike.

The system 100 comprises the identification codes 55 positioned on the materials 50, the identification devices 12 on board the trolleys 10, and the fixed identification members 203.

In some embodiments, the system 100 also comprises the identification codes 204 disposed in predetermined positions of the healthcare facility to identify the position of the trolley 10 and the telemetry devices 23 on board the trolleys 10.

In some embodiments, the system 100 also comprises the identification devices 19 for identifying the operators and the identification codes (badges) assigned to the operators 300 for their recognition.

The system 100 can comprise containing elements 205, such as baskets, worktops, trays or suchlike, for positioning the materials 50.

The containing elements 205 can be positioned in the operating theaters 200 and can comprise identification devices 203 for identifying the identification codes 55 located on the materials 50. For example, the labels 56 of the materials 50, or the materials 50 actually used, can be inserted in the containing elements 205. Advantageously, in this way it is possible to automatically generate the unloading lists of the materials used, for example identifying the fact that, after use, they have been thrown away, if disposable, or sent for sterilization.

The materials 50 not identified in this step can be recorded as "unused" (returned) and can be automatically unlinked from the operation.

The system 100 can also comprise loading and unloading gates 206, for example gates, platforms or suchlike, each comprising at least one fixed identification member 203 for identifying the identification codes 55 and which can be positioned both in the warehouses 201, 210 and also in proximity to the access point of an operating theater 200 for the automatic loading and unloading of the materials 50.

Advantageously, the use of the loading and unloading gates 206 can allow to perform the loading and unloading of the materials 50 in/from the warehouse 201 without human supervision. Indeed, in this case, when a trolley 10 passes under a loading and unloading gate 206, the fixed identification member 203 associated with it automatically reads the identification codes 55 associated with the materials 50 present in the trolley 10 and communicates this information to the central control unit 101. In this way, it is possible to track the materials 50 in order to know their position in real time, while keeping the stocks of the warehouses 201, 210, 211 updated.

The fixed identification members 203 can also be configured to read the identification codes of the operators 300, in order to keep the traceability of the operator 300 preparing the trolley 10. Alternatively, the loading and unloading gates 206 may have identification devices dedicated to reading the identification codes of the operators 300, not shown in the drawings.

The system 100 can comprise portable electronic devices 301, such as tablets, cell phones, smart watches, applications to be installed on personal devices or suchlike, to be assigned to the operators 300 and communicating with the communication devices 102 in order to send and receive data and information relating to the trolley 10 and/or information relating to the healthcare facility.

Advantageously, the operator 300 can be updated, on a portable device 301 allocated to him/her, on the status of the trolley 10, for example with information relating to the stocks of the materials 50, the data on the position of the trolley 10, the availability of materials 50 in the healthcare facility, for example in the warehouse 201, in the sterilization station and/or suchlike, the scheduling of the surgical operations and/or suchlike.

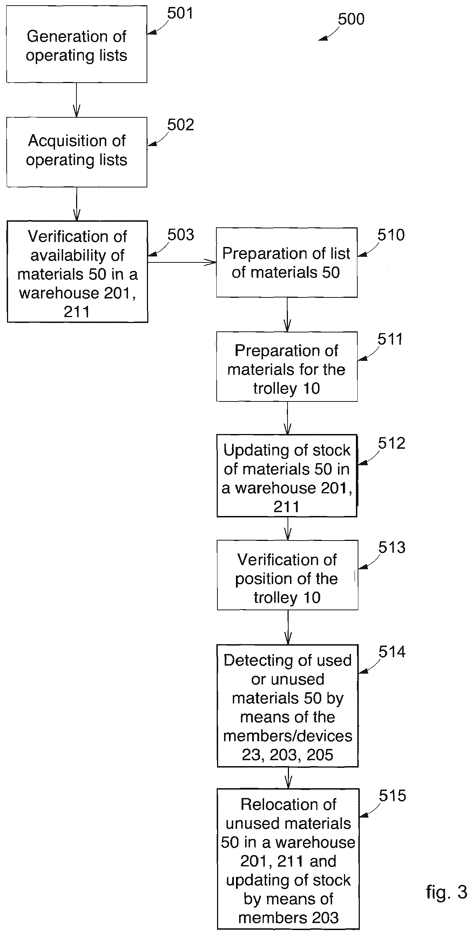

Some embodiments described here and shown by way of example in fig. 3 concern a method 500 for the automatic management of surgical materials 50 in a healthcare facility by means of a system 100 according to the invention, comprising, by way of example, the following steps:
- step 501: generation of the operating lists of the operations scheduled for a pre-established period of time;
- step 502: acquisition of the operating lists by the central control unit 101;
- step 503: verification of the availability of the materials 50 in the warehouses 201 and 211, by comparing the number of each material required on the basis of the operating lists generated with the stock of the various materials which has been stored;
- step 510: preparation of the list of materials 50 to be loaded onto a trolley 10, this list is communicated to an operator, for example displayed on the device 301 allocated to the operator which can also be identified;
- step 511: preparation of the materials for the trolley 10, removing them from the warehouse 201, 211 and taking them to the loading warehouse 210;
- step 512: upon leaving the warehouse 201, 211 updating of the stock of materials 50, by subtracting the removed quantities from the stock previously stored, preferably by automatically reading identification codes 55 located on the materials 50 by means of the fixed identification members 203;
- step 513: possible verification of the position of the trolley 10, in order to ascertain that it has actually been taken to the expected position, through the interaction between the telemetry device 23 and the identification codes 204;
- step 514: at the end of the pre-established time period, for example a whole day, detecting which materials 50 have been used and/or have remained unused by means of the fixed identification members 203 and/or the containing elements 205 disposed in the operating theaters 200, and communicating the information to the central control unit 101;
- step 515: updating of the stock of the warehouses 201, 211 in order to add the quantities of materials 50 which have remained unused to the current stock; preferably this occurs automatically by means of the fixed identification members 203 when the materials 50 are returned to the warehouse 201, 211.

The method 500 can provide to communicate any discrepancies/anomalies to an operator 300 and/or to a supervisor.

It can also be provided that the central control unit 101 of the system 100 communicates to the operator 300 which materials are left, for example by means of the portable electronic device 301 allocated to the operator.

The method 500 can provide in particular to:
- identify the loading and unloading of materials 50 in/from the trolley 10 by means of an identification device 12 for identifying the materials 50 which is comprised in the trolley 10;
- performing at least the calculation of the stocks of materials 50 left in the trolley 10 by means of the processing unit 13; and
- communicating the detected data to the central control unit 101 of the system 100.

The method 500 can provide that the information on the stocks of the materials 50 in the trolley 10 can be used to manage the restock of the materials 50 in the trolley 10.

The method 500 can provide to send the position data of the trolley 10 to a user device 301 of an operator 300. Advantageously, the trolley 10 can therefore allow the operator 300 to track the trolley 10 within the healthcare facility.

The method 500 can provide that the central control unit 101 communicates, by means of the communication devices 102, with the at least one trolley 10, detecting at least information relating to the stocks of the materials 50 in the trolley 10.

The method 500 can provide that central units of the healthcare facility manage the surgical scheduling of the operating blocks, for example by generating 501 the daily lists of the operations of the operating sessions for each block.

The method can therefore provide to carry out, for each surgical operation, or for each group of surgical operations, the steps 510 to 513 described above.

It is clear that modifications and/or additions of parts and/or steps may be made to the surgical trolley 10, to the system 100 and to the method 500 as described heretofore, without departing from the field and scope of the present invention, as defined by the claims.

It is also clear that, although the present invention has been described with reference to some specific examples, a person of skill in the art will be able to achieve other equivalent forms of system and method for the automatic management and tracking of surgical materials, having the characteristics as set forth in the claims and hence all coming within the field of protection defined thereby.

In the following claims, the sole purpose of the references in brackets is to facilitate their reading and they must not be considered as restrictive factors with regard to the field of protection defined by the same claims.

## Claims

1. Method for the automatic management and tracking of surgical or medical materials (50), **characterized in that** it comprises the steps of:
- receiving a list of materials (50) needed on the basis of the scheduling of one or more surgical operations of at least one operating theater (200) in a pre-established time interval;
- through the automatic reading of identification codes (55) located on said materials (50), verifying their removal from a materials warehouse (201) and possibly from a pharmaceutical warehouse (211) and subsequent placement in a mobile trolley (10) dedicated to said operating theater (200);
- automatically updating the stock of said materials (50) in said materials warehouse (201), by subtracting the quantities of removed materials from the current stock value in order to calculate a current stock;
- once said time interval has elapsed, detecting, through identification means (12, 203) provided on board said trolley (10) and/or in said operating theater (200), which materials (50) located in said trolley (10) have remained unused;
- automatically updating the stock of said materials (50) in said materials warehouse (201) by adding to the current stock the quantities of unused materials after the latter have been detected by additional identification means (203) present in said materials warehouse (201).

2. Method as in claim 1, **characterized in that** it comprises a step of verifying the position of said trolley (10) inside said healthcare facility through the interaction between a telemetry device (23) on board said trolley (10) and identification codes (204) placed in predetermined positions in the healthcare facility.

3. Method as in claim 1 or 2, **characterized in that** it comprises a step of identifying an operator (300) able to manipulate said materials (50) for their removal from/deposit into said trolley (10), or said materials warehouse (201), or said operating theater (200).

4. Method as in any claim hereinbefore, **characterized in that** the steps of claim 1 are repeated also for drugs or medicaments stored in a pharmaceutical warehouse (211).

5. Method as in claim 4, **characterized in that** it comprises a step of loading said trolley (10) with said materials (50) by an operator in a loading warehouse (210), to which said materials (50) arrive coming from said materials warehouse (201) and/or from said pharmaceutical warehouse (211).

6. System (100) for the automatic management and tracking of surgical materials (50) in a healthcare facility comprising one or more operating theaters (200), the system (100) is **characterized in that** it comprises:
- one or more surgical trolleys (10) configured to receive a plurality of surgical or medical materials (50) and mobile in order to reach said operating theaters (200),
- identification codes (55) located on said materials (50),
- identification means (12, 203) placed on board each of said trolleys (10), in each operating theater (200), in a materials warehouse (201) and possibly in a pharmaceutical warehouse (211) where said materials (50) are stored,
- a central control unit (101) configured to process the data received from said identification means (12, 203) in order to update the stock of materials (50) present in said materials warehouse (201), also considering materials (50) removed from the latter, placed in said trolleys (10) and left unused at the end of a pre-established time interval.

7. System (100) as in claim 6, **characterized in that** it comprises identification codes (204) placed in predetermined positions of the healthcare facility, and a telemetry device (23) configured to be disposed in each of said trolleys (10) in order to interact with said identification codes (204) and detect the position of said trolleys (10) in said healthcare facility.

8. System (100) as in claim 6 or 7, **characterized in that** it comprises loading and unloading gates (206) disposed in one or more of said operating theaters (200) and/or in said materials warehouse (201), wherein with each of said loading and unloading gates (206) there is associated a respective fixed identification member (203) comprised in said identification means (12, 203) to automatically load or unload the materials (50) from the stock of the materials present in said operating theaters (200) and/or in said materials warehouse (201).

9. System (100) as in any claim from 6 to 8, **characterized in that** it comprises elements (205) for containing said materials (50), positioned in said operating theaters (200) and comprising fixed identification members (203) comprised in said identification means (12, 203).

10. System (100) as in any claim from 6 to 9, **characterized in that** it comprises communication devices (102) for communicating between said central control unit (101) and said identification means (12, 203), said communication devices (102) being configured to also communicate with electronic devices (301) allocated to the operators (300) and possibly with one or more central units of said healthcare facility.

11. System (100) as in claim 10, **characterized in that** each of said trolleys (10) comprises a communication device (14) configured to interface with said communication devices (102) in order to communicate with said central control unit (101), external with respect to said trolley (10), wherein said communication device (14) is configured for the automatic and real-time communication at pre-established time frequencies.

12. System (100) as in any claim from 6 to 11, **characterized in that** each of said trolleys (10) comprises a detection device (19) configured to detect the identity of an operator (300).

13. System (100) as in any claim from 6 to 12, **characterized in that** each of said trolleys (10) comprises an electric power supply device (24).

14. System (100) as in any claim from 6 to 13, **characterized in that** each of said trolleys (10) comprises a processing unit (13) comprising at least one management algorithm configured to execute at least the calculation of the stocks of said materials (50) on the basis of the materials (50) actually loaded into or unloaded from said trolley (10).

15. System (100) as in claim 14, **characterized in that** each of said trolleys (10) comprises one or more detection devices (25) selected from temperature sensors, speed sensors, displacement sensors, shock detectors, and said processing unit (13) is configured to collect, store and process the data measured by said detection devices (25).
